# DEMANDE DE BREVET EUROPEEN

(11) **EP 1 804 061 A1**
(43) Date de publication de la demande: **04.07.2007**
(21) Numéro de dépôt: 06292021.0
(22) Date de dépôt: 22.12.2006
(51) Int. Cl.: G01N 33/569, G01N 33/68, A61K 38/16

(54) **Utilisation de la protéine 5E2 dans le diagnostic in vitro d'infections à Legionella pneumophila**

(30) Priorité: 28.12.2005 FR 0513416
(71) Demandeur: ABAG, 91380 Chilly Mazarin (FR); UNIVERSITE CLAUDE BERNARD - LYON 1, 69100 Villeurbanne (FR)
(72) Inventeur: Arie, Jean-Philippe, 75013 Paris (FR); Cyncynatus, Camille, 94410 Saint Maurice (FR)
(74) Mandataire: Sauvage, Renée

(57) **Abrégé**

L'invention a pour objet l'utilisation de la protéine 5E2, de séquence polypeptidique ID SEQ N°2, ou des fragments ou des variants de ladite protéine, l'utilisation des séquences codant pour lesdites protéines et l'utilisation d'anticorps dirigés contre lesdites protéines dans le domaine du diagnostic *in vitro* d'une infection à *Legionella pneumophila.*

LISTAGE DE SEQUENCES

<110>  ABAG
       Université Claude Bernard Lyon 1

<120>  Utilisation de la protéine 5E2 dans le diagnostic in vitro
       d'infection à Legionella pneumophila

<130>  B4210

<150>  FR 05 13416
<151>  2005-12-28

<160>  2

<170>  PatentIn version 3.3

<210>  1
<211>  1224
<212>  DNA
<213>  Legionella pneumophila


<220>
<221>  CDS
<222>  (1)..(1224)

<400>  1
tct att gaa gtc aaa gta cct gtt cta ccc gag tca gta gct gat gca        48
Ser Ile Glu Val Lys Val Pro Val Leu Pro Glu Ser Val Ala Asp Ala
1               5                   10                  15

aca gtg gct gcg tgg cat aag aaa gtg ggt gat aaa gta tct cgc gat        96
Thr Val Ala Ala Trp His Lys Lys Val Gly Asp Lys Val Ser Arg Asp
            20                  25                  30

gag aac cta gtg gat ctg gaa aca gat aaa gtt gtg ctt gaa gtt cca       144
Glu Asn Leu Val Asp Leu Glu Thr Asp Lys Val Val Leu Glu Val Pro
        35                  40                  45

tca cct gtg gat ggg gta ttg tct gac att ctg ttt aat acc gga gat       192
Ser Pro Val Asp Gly Val Leu Ser Asp Ile Leu Phe Asn Thr Gly Asp
    50                  55                  60

acg gtt ggt tca gga gac tta ctg gca aaa att tct caa agt gtt tct       240
Thr Val Gly Ser Gly Asp Leu Leu Ala Lys Ile Ser Gln Ser Val Ser
65                  70                  75                  80

gta gaa tct caa aaa act gag aaa gaa gag aaa cct gtc aaa aaa gaa       288
Val Glu Ser Gln Lys Thr Glu Lys Glu Glu Lys Pro Val Lys Lys Glu
                85                  90                  95

gaa ata aaa att tcc gaa agt gaa tca gtt agc aca aaa gaa gat aaa       336
Glu Ile Lys Ile Ser Glu Ser Glu Ser Val Ser Thr Lys Glu Asp Lys
            100                 105                 110

tca acc agt ccg gta gta aga cgg atg atg gct gaa aat gat ttg aaa       384
Ser Thr Ser Pro Val Val Arg Arg Met Met Ala Glu Asn Asp Leu Lys
        115                 120                 125

gcc ggg cag att caa ggt acc ggt aaa gat ggc agg ata acc aaa gaa     432
Ala Gly Gln Ile Gln Gly Thr Gly Lys Asp Gly Arg Ile Thr Lys Glu
    130                 135                 140

gac gtt tta tct tat ata gaa tct aat aga gaa aaa gca aac caa acc     480
Asp Val Leu Ser Tyr Ile Glu Ser Asn Arg Glu Lys Ala Asn Gln Thr
145                 150                 155                 160

gct gaa gtt caa aaa gaa cct tct atg aga gca ctt tcc ttt ggc ggc     528
Ala Glu Val Gln Lys Glu Pro Ser Met Arg Ala Leu Ser Phe Gly Gly
                165                 170                 175

agg gaa gaa aaa cgt gtc cct atg acg aga cta aga gcc aag att gcg     576
Arg Glu Glu Lys Arg Val Pro Met Thr Arg Leu Arg Ala Lys Ile Ala
            180                 185                 190

gaa cgt tta cta caa gca caa cac aac gct gcc atg tta acc act ttt     624
Glu Arg Leu Leu Gln Ala Gln His Asn Ala Ala Met Leu Thr Thr Phe
        195                 200                 205

aac gaa gtg aat ttg aag gca gtt atg gac atg cgt tct cag tat aag     672
Asn Glu Val Asn Leu Lys Ala Val Met Asp Met Arg Ser Gln Tyr Lys
    210                 215                 220

gat agt ttt gaa aaa aaa cat ggt gta aaa ctt gga ttt atg tct ttt     720
Asp Ser Phe Glu Lys Lys His Gly Val Lys Leu Gly Phe Met Ser Phe
225                 230                 235                 240

ttc aca aaa gca gta gta gag tcg ctg aaa cga ttc ccg gcc gtg aat     768
Phe Thr Lys Ala Val Val Glu Ser Leu Lys Arg Phe Pro Ala Val Asn
                245                 250                 255

gct tcc att gat ggg caa gat att gtt tat cat ggt ttt tat gat ata     816
Ala Ser Ile Asp Gly Gln Asp Ile Val Tyr His Gly Phe Tyr Asp Ile
            260                 265                 270

gga att gct gtt tct act gaa aga ggt tta gtt gtt ccg gta att aga     864
Gly Ile Ala Val Ser Thr Glu Arg Gly Leu Val Val Pro Val Ile Arg
        275                 280                 285

gac gct gat caa tta agc atg gca gaa att gaa tta gcc att aat gat     912
Asp Ala Asp Gln Leu Ser Met Ala Glu Ile Glu Leu Ala Ile Asn Asp
    290                 295                 300

gcg gca tca aaa gcc aga acc ggt aag ctg tct atg gaa gag atg caa     960
Ala Ala Ser Lys Ala Arg Thr Gly Lys Leu Ser Met Glu Glu Met Gln
305                 310                 315                 320

ggg gga aca ttt acc ata acc aac ggt gga gta ttc ggt tca tta tta    1008
Gly Gly Thr Phe Thr Ile Thr Asn Gly Gly Val Phe Gly Ser Leu Leu
                325                 330                 335

gct act ccc att atc aac cca ccg caa aca ggt atc ttg ggg atg cat    1056
Ala Thr Pro Ile Ile Asn Pro Pro Gln Thr Gly Ile Leu Gly Met His
            340                 345                 350

aaa att gag gaa aga cct att gta gag aaa ggg caa ata gta atc cgt    1104
Lys Ile Glu Glu Arg Pro Ile Val Glu Lys Gly Gln Ile Val Ile Arg
        355                 360                 365

cct atg atg tat gtt gct ttg tct tat gat cat cgt tta att gat gga     1152
Pro Met Met Tyr Val Ala Leu Ser Tyr Asp His Arg Leu Ile Asp Gly
    370                 375                 380

aaa gat tct gta caa ttt tta gta agt gtt aaa gag ttg ttg gaa gat     1200
Lys Asp Ser Val Gln Phe Leu Val Ser Val Lys Glu Leu Leu Glu Asp
385                 390                 395                 400

ccg tct cga ctt tta ctt aat gtt                                     1224
Pro Ser Arg Leu Leu Leu Asn Val
                405

<210>  2
<211>  408
<212>  PRT
<213>  Legionella pneumophila

<400>  2

Ser Ile Glu Val Lys Val Pro Val Leu Pro Glu Ser Val Ala Asp Ala
1               5                   10                  15

Thr Val Ala Ala Trp His Lys Lys Val Gly Asp Lys Val Ser Arg Asp
            20                  25                  30

Glu Asn Leu Val Asp Leu Glu Thr Asp Lys Val Val Leu Glu Val Pro
        35                  40                  45

Ser Pro Val Asp Gly Val Leu Ser Asp Ile Leu Phe Asn Thr Gly Asp
    50                  55                  60

Thr Val Gly Ser Gly Asp Leu Leu Ala Lys Ile Ser Gln Ser Val Ser
65                  70                  75                  80

Val Glu Ser Gln Lys Thr Glu Lys Glu Glu Lys Pro Val Lys Lys Glu
                85                  90                  95

Glu Ile Lys Ile Ser Glu Ser Glu Ser Val Ser Thr Lys Glu Asp Lys
            100                 105                 110

Ser Thr Ser Pro Val Val Arg Arg Met Met Ala Glu Asn Asp Leu Lys
        115                 120                 125

Ala Gly Gln Ile Gln Gly Thr Gly Lys Asp Gly Arg Ile Thr Lys Glu
    130                 135                 140

Asp Val Leu Ser Tyr Ile Glu Ser Asn Arg Glu Lys Ala Asn Gln Thr
145                 150                 155                 160

Ala Glu Val Gln Lys Glu Pro Ser Met Arg Ala Leu Ser Phe Gly Gly
                165                 170                 175

Arg Glu Glu Lys Arg Val Pro Met Thr Arg Leu Arg Ala Lys Ile Ala
            180                 185                 190

Glu Arg Leu Leu Gln Ala Gln His Asn Ala Ala Met Leu Thr Thr Phe
        195                 200                 205

Asn Glu Val Asn Leu Lys Ala Val Met Asp Met Arg Ser Gln Tyr Lys
    210                 215                 220

Asp Ser Phe Glu Lys Lys His Gly Val Lys Leu Gly Phe Met Ser Phe
225                 230                 235                 240

Phe Thr Lys Ala Val Val Glu Ser Leu Lys Arg Phe Pro Ala Val Asn
                245                 250                 255

Ala Ser Ile Asp Gly Gln Asp Ile Val Tyr His Gly Phe Tyr Asp Ile
            260                 265                 270

Gly Ile Ala Val Ser Thr Glu Arg Gly Leu Val Val Pro Val Ile Arg
        275                 280                 285

Asp Ala Asp Gln Leu Ser Met Ala Glu Ile Glu Leu Ala Ile Asn Asp
    290                 295                 300

Ala Ala Ser Lys Ala Arg Thr Gly Lys Leu Ser Met Glu Glu Met Gln
305                 310                 315                 320

Gly Gly Thr Phe Thr Ile Thr Asn Gly Gly Val Phe Gly Ser Leu Leu
                325                 330                 335

Ala Thr Pro Ile Ile Asn Pro Pro Gln Thr Gly Ile Leu Gly Met His
            340                 345                 350

Lys Ile Glu Glu Arg Pro Ile Val Glu Lys Gly Gln Ile Val Ile Arg
        355                 360                 365

Pro Met Met Tyr Val Ala Leu Ser Tyr Asp His Arg Leu Ile Asp Gly
    370                 375                 380

Lys Asp Ser Val Gln Phe Leu Val Ser Val Lys Glu Leu Leu Glu Asp
385                 390                 395                 400

Pro Ser Arg Leu Leu Leu Asn Val
                405

## Description

La présente invention concerne les infections à *Legionella pneumophila.*

Plus précisément, l'invention porte sur l'utilisation d'un polypeptide nouvellement sélectionné, d'un polynucléotide, d'un anticorps correspondant, d'une part, dans le domaine du diagnostic sérologique des infections impliquant *Legionella pneumophila* et, d'autre part, dans le domaine de la production de vaccins vis-à-vis de cette bactérie.

Les légionelloses sont des infections respiratoires sévères résultant de l'inhalation d'aérosols contaminés par des bactéries à croissance intracellulaire du genre Legionella, et qui se manifestent sous forme sporadique ou épidémique. Ces infections surviennent aussi bien en ville (infections communautaires) qu'en milieu hospitalier (infections nosocomiales). L'espèce *Legionella pneumophila* est responsable de 95% de l'ensemble des cas de légionellose.

La légionellose, infection pulmonaire très souvent aiguë, se caractérise par la sévérité du tableau clinique : après une incubation de 2 à 10 jours et un début pseudo-grippal, le malade présente une température élevée, un malaise général, des douleurs abdominales, voire des troubles psychiques. La radiologie révèle que l'atteinte est souvent bilatérale. La mortalité associée, particulièrement élevée chez certaines personnes dites "à risque" (sujets âgés ou souffrant d'un cancer, d'une hémopathie), varie de 10 à 20% (13% des 835 cas recensés en France en 2002).

Du fait de l'enjeu thérapeutique, il est essentiel de pouvoir poser précocement le diagnostic de légionellose. En effet, les légionelles sont insensibles aux bêta-lactamines - qui sont les principaux antibiotiques prescrits en cas de pneumopathie - et il faut avoir recours à un traitement spécifique reposant sur les macrolides (érythromycine) ou les fluoroquinolones.

A l'heure actuelle, le diagnostic est réalisé par trois méthodes générales : la mise en évidence des bactéries à partir de prélèvements broncho-pulmonaires (soit directement par immunofluorescence soit par culture sur milieux sélectifs) ; la détection d'antigènes urinaires spécifiques ; la mise en évidence d'une augmentation significative du titre des anticorps dans le sérum (sérologie).

L'examen direct des prélèvements réalisé par microscopie à fluorescence se pratique, pour la majorité des réactifs commercialisés, à l'aide d'anticorps monoclonaux ou polyclonaux qui reconnaissent l'ensemble des sérogroupes connus de *L. pneumophila.* Le principal inconvénient de cette technique est sa faible sensibilité. Sa spécificité est également imparfaite (environ 90%), du fait de réactions immunologiques croisées avec certaines bactéries comme *Pseudomonas aeruginosa, Bordetella pertussis* ou *Bacteroides fragilis.* La culture sur milieux spécifiques est la méthode de choix, notamment dans un but épidémiologique (typage des souches). Elle donne toutefois des résultats tardifs puisque les légionelles cultivent en 3 à 4 jours, voire plus. La technique d'immunofluorescence indirecte (IFI) est la technique la plus employée pour le diagnostic sérologique **(**McDade JE, Shepard CC, Fraser DW, Tsai TR, Redus MA et Dowdle WR (1977), Legionnaires' disease : isolation of a bacterium and demonstration of its role in other respiratory disease, N Engl J Med. 297 (22) : 1197-203**).** Environ 70% des diagnostics au niveau européen sont réalisés par cette méthode.

Les tests urinaires sont rapides et très spécifiques. Néanmoins, leur sensibilité est très variable, de 50-90% ; cette forte variabilité est liée non seulement aux caractéristiques du patient mais aussi au fait que les tests actuels ne permettent que la détection de *L. pneumophila* de sérogroupe 1. De plus, la cinétique d'apparition et de disparition des antigènes de *Legionella* dans les urines est aussi très variable selon les patients. Un test positif n'est ainsi pas le signe d'une légionellose en phase aiguë et la rechute ou la récidive ne peut être évaluée par cette méthode. Enfin, en présence d'une recherche positive d'antigènes urinaires de légionelles, il est nécessaire de réaliser en parallèle, une culture de prélèvements respiratoires pour l'isolement de la bactérie dans un but épidémiologique.

Le sérodiagnostic est tout particulièrement utile chez les patients qui ne produisent pas d'expectoration et en cas d'enquête épidémiologique. Il repose sur la mise en évidence d'une augmentation significative du taux des anticorps entre un premier sérum et un sérum prélevé 4 à 6 semaines plus tard. Environ 30% des cas de légionellose sont identifiés par cette méthode à l'heure actuelle. Néanmoins, les antigènes utilisés dans les tests actuels sont constitués de simples extraits préparés directement à partir de culture de légionelles inactivées par la chaleur ou après ensemencement des bactéries dans le sac vitellin d'oeufs embryonnés. Les anticorps détectés reconnaissent en majorité des déterminants du lipopolysaccharide (LPS), qui peuvent être proches de déterminants du LPS d'autres bactéries et provoquer des réactions croisées. De fait, des réactions de ce type ont été décrites avec d'autres bactéries à Gram négatif telles que *Pseudomonas sp., Bacteroides sp., Bordetella sp.* et certaines entérobactéries. Enfin, la plupart des tests actuels permettent seulement la détection d'anticorps de type IgG, ce qui nécessite deux prises de sang à 4-6 semaines d'intervalle pour faire un diagnostic d'infection récente.

En résumé, les pratiques courantes répondent imparfaitement aux attentes médicales pour établir le diagnostic de légionellose. En particulier, les tests sérologiques actuels reposent sur des méthodes de détection anciennes, non automatisables, ou qui utilisent des antigènes non caractérisés ("soupes antigéniques"), dont la spécificité et la sensibilité sont peu satisfaisantes. L'utilisation de la sérologie souffre de très grandes variations selon les malades et les tests basés sur la lecture d'un titre unique précoce possèdent des valeurs prédictives positives très faibles de l'ordre de 10-15% **(**Jarraud S, Reyrolle M et Etienne J. dans Freney J, Renaud F, Hansen W, Bollet C, Précis de bactériologie clinique, ed. ESKA, Paris, 2000**).**

Il existe donc un très grand besoin, dans le domaine du diagnostic sérologique, en un moyen permettant de réaliser des tests rapides, peu coûteux et non invasifs, et qui pourraient également permettre un diagnostic plus précoce. Enfin, dans l'avenir, en cas de développement d'approches vaccinales visant à prévenir les infections à *Legionella pneumophila,* de tels tests seront indispensables pour détecter les échecs de vaccination.

Les inventeurs de la présente invention ont identifié de façon tout à fait inattendue à partir du génome de *Legionella pneumophila,* un polypeptide qu'ils ont appelé 5E2 de séquence polypeptidique ID SEQ N°2. L'identification de ce polypeptide est le résultat d'un crible et d'études approfondies que les séquences issues des programmes de génomique de *L. pneumophila* ne laissaient pas envisager. Le polypeptide a été sélectionné, spécifiquement pour ses propriétés antigéniques, parmi nombre d'autres issus de *L. pneumophila,* dont certains localisés à la surface de la bactérie ou intrinsèques à la membrane et qui se sont avérés non pertinents pour le diagnostic sérologique d'une telle infection. Par exemple, la Déposante connaît des polypeptides issus de *L. pneumophila,* comme les protéines OmpS, Mip, et certains composants des systèmes de sécrétion Lsp et Dot/Icm tels que LspI, LspJ, LspK, IcmJ et IcmF, recombinants chez *E. coli,* ou d'autres encore, qui ne permettent pas de diagnostiquer par sérologie une infection à *Legionella pneumophila.*

Les utilisations possibles du polypeptide 5E2 sont décrites ci-après.

La présente invention a ainsi, notamment, comme objectifs de résoudre les déficiences des tests sérologiques actuels et de permettre une analyse plus précise et plus complète de l'infection grâce à la détection des différents isotypes d'anticorps spécifiques du polypeptide et, éventuellement, de diagnostiquer différents sérogroupes et espèces de légionelles.

### Définitions

Les définitions suivantes sont données afin de faciliter la compréhension de certains termes utilisés dans cette description.

On entend par "polynucléotide" un polyribonucléotide ou un polydésoxyribonucléotide qui peut être un ADN ou un ARN, modifié ou non.

Le terme polynucléotide inclut, sans limitation, un ADN simple brin ou double brin, un ADN composé d'un mélange d'une ou plusieurs région(s) simple brin et d'une ou plusieurs région(s) double brin, un ADN qui est un mélange de régions simple brin, double brin et/ou triple brin, un ARN simple brin ou double brin, un ARN composé d'un mélange d'une ou plusieurs région(s) simple brin et d'une ou plusieurs région(s) double brin et les molécules hybrides comprenant un ADN et un ARN qui peuvent comprendre des régions simple brin, double brin et/ou triple brin ou un mélange de régions simple brin et double brin. Le terme polynucléotide peut aussi comprendre un ARN et/ou un ADN comprenant une ou plusieurs régions triple brin. Les brins, dans de telles régions, peuvent provenir de la même molécule ou de molécules différentes. Par conséquent, les ADN ou ARN, avec des squelettes modifiés pour la stabilité ou d'autres raisons, sont inclus dans le terme polynucléotides. On entend également, par polynucléotide, les ADN et ARN contenant une ou plusieurs bases modifiées. On entend par base modifiée, par exemple, les bases inhabituelles telles que l'inosine. Le terme polynucléotide vise également les polynucléotides de forme modifiée chimiquement, enzymatiquement ou métaboliquement. Les polynucléotides comprennent également les polynucléotides courts tels que les oligonucléotides.

On entend par "polypeptide" un peptide, un oligopeptide, un oligomère ou une protéine comprenant au moins deux acides aminés joints l'un à l'autre par une liaison peptidique normale ou modifiée.

Le terme polypeptide comprend les chaînes courtes, appelées peptides, oligopeptides et oligomères, et les chaînes longues, appelées protéines.

Un polypeptide peut être composé d'acides aminés autres que les 20 acides aminés codés par les gènes humains. Un polypeptide peut également être composé d'acides aminés modifiés par des processus naturels, tels que le processus de maturation post-traductionnel ou par des procédés chimiques, qui sont bien connus de l'homme du métier. Le même type de modification peut être présent à plusieurs endroits du polypeptide et n'importe où dans le polypeptide : dans le squelette peptidique, dans la chaîne d'acides aminés ou encore aux extrémités carboxy- ou amino-terminales.

Un polypeptide peut être ramifié suite à une ubiquitination ou être cyclique, avec ou sans ramification. Ce type de modifications peut être le résultat de processus de post-traduction naturel ou synthétique, qui sont bien connus de l'homme du métier.

On entend, par exemple, par modifications d'un polypeptide, l'acétylation, l'acylation, l'ADP-ribosylation, l'amidation, la fixation covalente de flavine, la fixation covalente d'un hème, la fixation covalente d'un nucléotide ou d'un dérivé nucléotidique, la fixation covalente d'un lipide ou d'un dérivé lipidique, la fixation covalente d'un phosphatidylinositol, la réticulation covalente ou non-covalente, la cyclisation, la formation de pont disulfure, la déméthylation, la formation de cystéine, la formation de pyroglutamate, la formylation, la gamma-carboxylation, la glycosylation, la formation d'ancre au GPI, l'hydroxylation, l'iodisation, la méthylation, la myristoylation, l'oxydation, le processus protéolytique, la phosphorylation, la prénylation, la racémisation, la sénéloylation, la sulfatation, l'addition d'acides aminés telles que l'arginylation ou l'ubiquitination **(**PROTEINS STRUCTURE AND MOLECULAR PROPERTIES, 2nd Ed., T.E. Creighton, W.H. Freeman and Company, New York (1993**) and** Wold, F., Posttranslational Protein Modifications: Perspectives and Prospects, pgs 1-12 in POSTTRANSLATIONAL COVALENT MODIFICATION OF PROTEINS, B.C. Johnson, Ed., Academic Press, New York (1983**) ;** Seifter et al., Meth. Enzymol. 182:626-646 (1990) **and** Rattan et al., Protein Synthesis: Posttranslational Modifications and Aging, Ann. N.Y. Acad. Sci. 663:48-62 (1992)**).**

On entend par "pourcentage d'identité" entre deux séquences polynucléotidiques ou polypeptidiques le pourcentage de nucléotides ou d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons entre deux séquences polynucléotidiques ou polypeptidiques sont traditionnellement réalisées en comparant ces séquences après les avoir alignées de manière optimale, ladite comparaison étant réalisée par segment ou par "fenêtre de comparaison" pour identifier et comparer les régions locales de similarité de séquence. Cette comparaison peut être réalisée grâce à un programme, par exemple le programme EMBOSS-Needle (alignement global Needleman-Wunsch) à l'aide de la matrice BLOSUM62/Open Gap 10.0 et Extension Penalty de 0,5 **(**Needleman et Wunsch (1970). J. Mol. Biol. 48, 443-453 **et** Kruskal, J.B. (1983), An overview of sequence comparison, In D. Sankoff and J.B. Kruskal, (ed), Time warps, strind edits and macromolecules : the theory and practice of sequence comparison, pp. 1-44 Addison Wesley**).**

Le pourcentage d'identité est calculé en déterminant le nombre de positions identiques pour lesquelles le nucléotide ou l'acide aminé est identique entre les deux séquences, en divisant ce nombre de positions identiques par le nombre total de positions dans la fenêtre de comparaison et en multipliant le résultat obtenu par 100.

Un polynucléotide ayant, par exemple, une identité d'au moins 95% avec le polynucléotide ID SEQ N°1 est donc un polynucléotide comportant, au plus, 5 nucléotides modifiés sur 100 nucléotides, par rapport à ladite séquence. En d'autres termes jusqu'à 5% des nucléotides de la séquence ID SEQ N°1 peuvent être délétés ou substitués par un autre nucléotide, ou jusqu'à 5% du nombre total de nucléotides de la séquence ID SEQ N°1 peuvent être insérés dans ladite séquence. Ces modifications peuvent être positionnées aux extrémités 3' et/ou 5', ou à un endroit quelconque entre ces extrémités, en un seul ou plusieurs emplacements.

Par analogie, un polypeptide ayant, par exemple, une identité d'au moins 95% avec le polypeptide ID SEQ N°2 est un polypeptide comportant, au plus, 5 acides aminés modifiés sur 100 acides aminés, par rapport à ladite séquence. En d'autres termes jusqu'à 5% des acides aminés dans la séquence ID SEQ N°2 peuvent être délétés ou substitués par un autre acide aminé ou jusqu'à 5% du nombre total d'acides aminés de la séquence ID SEQ N°2 peuvent être insérés dans ladite séquence. Ces altérations de la séquence peuvent être situées aux positions amino et/ou carboxy-terminales de la séquence d'acides aminés ou à un endroit quelconque entre ces positions terminales, en un ou plusieurs emplacements **(**Computational Molecular Biology, Lesk, A.M., ed., Oxford University Press, New York, 1988**;** Biocomputing: Informatics and Genome Projects, Smith, D.W., ed., Academic Press, New York, 1993**;** Computer Analysis of Sequence Data, Part I, Griffin, A.M., and Griffin, H.G., eds., Humana Press, New Jersey, 1994 **;** Sequence Analysis in Molecular Biology, von Heinje, G., Academic Press, 1987**).**

La "similarité" est, quant à elle, calculée de la même façon que l'identité, excepté que les acides aminés non identiques, mais présentant des caractéristiques physico-chimiques communes, sont considérés comme identiques.

On entend par "fragment de polypeptide" un polypeptide comprenant au moins "n" acides aminés consécutifs issus du polypeptide ID SEQ N°2, où, selon les séquences, n sera égal à 7 acides aminés ou plus (par exemple, 8, 10, 12, 14, 16, 18, 20, 30, 40, 50 ou plus acides aminés). Préférentiellement, lesdits fragments comportent un épitope de la séquence ID SEQ N°2. Ces épitopes de type B ou T peuvent être déterminés par un logiciel (par exemple, PEOPLE [Alix, 1999], PREDITOP [Pellequer et Westhof, 1993] ou TEST [Zao et al., 2001]) ou expérimentalement par des techniques classiques [par exemple, epitope mapping (cartographie d'épitope) ou protéolyse ménagée].

On entend par "fragment de polynucléotide" un polynucléotide comprenant au moins "n" nucléotides consécutifs issus du polynucléotide ID SEQ N°1, où, selon les séquences, n sera égal à 21 nucléotides ou plus (par exemple, 22, 23, 24, 25, 30, 36, 42, 48, 54, 60, 90, 120, 150 ou plus nucléotides).

On entend par "cellule hôte" une cellule qui a été transformée ou transfectée, ou qui est capable de transformation ou de transfection, par une séquence polynucléotidique exogène.

On entend par "milieu de culture" le milieu dans lequel on purifie le polypeptide de l'invention. Ce milieu peut être constitué par le milieu extracellulaire et/ou le lysat cellulaire. Des techniques bien connues de l'homme du métier permettent également à ce dernier de redonner la conformation active au polypeptide, si la conformation dudit polypeptide a été altérée lors de l'isolation ou de la purification.

On entend par "fonction" l'activité biologique d'un polypeptide ou d'un polynucléotide.

La fonction du polypeptide conforme à l'invention est celle d'un antigène de *Legionella pneumophila,* et la fonction du polynucléotide conforme à l'invention est celle de coder ce polypeptide. La fonction d'un antigène selon l'invention est de permettre d'établir un diagnostic d'une infection à *Legionella pneumophila,* mais aussi de réaliser des suivis thérapeutiques et, enfin, de préciser s'il s'agit d'une infection active ou aiguë, par exemple, par l'utilisation de la détection de différents isotypes d'anticorps.

On entend par "antigène" tout composé qui, seul ou en association avec un adjuvant ou porteur, est capable d'induire une réponse immunitaire spécifique. Cette définition comprend également tout composé présentant une analogie structurale avec ledit antigène capable d'induire une réponse immunologique dirigée contre ledit antigène.

On entend par "analogie structurale" une analogie aussi bien de la structure primaire (séquence) que de la structure secondaire (éléments structuraux), de la structure tertiaire (structure tridimensionnelle) ou de la structure quaternaire (association de plusieurs polypeptides dans un même complexe) **(**BIOCHEMISTRY, 4ème Ed, L. Stryer, New York, 1995**).**

On entend par "variant" d'un polynucléotide dit initial ou d'un polypeptide dit initial, respectivement, un polynucléotide ou un polypeptide qui en diffère par au moins un nucléotide ou un acide aminé, mais qui garde, respectivement, les mêmes propriétés intrinsèques, c'est-à-dire la même fonction.

Une différence dans la séquence polynucléotidique du variant peut altérer ou non la séquence d'acides aminés du polypeptide qu'il code, par rapport à un polypeptide initial. Néanmoins, par définition, ces variants doivent conférer la même fonction que la séquence polynucléotidique initiale, par exemple, coder un polypeptide ayant une fonction antigénique.

Le polynucléotide ou le polypeptide variant diffère généralement, et respectivement, du polynucléotide initial ou du polypeptide initial par une (ou plusieurs) substitution(s), addition(s), délétion(s), fusion(s) ou troncation(s) ou plusieurs de ces modifications, prises en combinaison. Un variant non-naturel d'un polynucléotide initial ou d'un polypeptide initial peut être obtenu, par exemple, par mutagenèse dirigée ou par synthèse directe.

On définit comme "séquence polynucléotidique complémentaire à la séquence polynucléotidique" un polynucléotide qui peut être hybridé avec cette séquence polynucléotidique dans des conditions stringentes.

On entend généralement, mais pas nécessairement, par "conditions stringentes" les conditions chimiques qui permettent une hybridation lorsque les séquences polynucléotidiques ont une identité d'au moins 80%.

Ces conditions peuvent être obtenues selon les méthodes bien connues de l'homme du métier.

On entend par "anticorps" les anticorps monoclonaux, polyclonaux, chimériques, simple chaîne, humanisés, ainsi que les fragments Fab, incluant les produits d'un Fab ou d'une banque d'expression d'immunoglobulines. Un anticorps immunospécifique peut être obtenu par administration à un animal d'un polypeptide donné, suivie d'une récupération des anticorps produits par ledit animal par extraction depuis ses fluides corporels. On peut également administrer à l'animal un variant dudit polypeptide, ou des cellules hôtes exprimant ce polypeptide.

Le terme "immunospécifique" appliqué au terme anticorps, vis-à-vis d'un polypeptide donné, signifie que l'anticorps possède une meilleure affinité pour ce polypeptide que pour d'autres polypeptides connus de l'art antérieur.

Par "affinité" on entend à la fois une complémentarité structurale et une complémentarité électrostatique entre deux molécules au sens d'une définition communément admise (voir, par exemple, Klotz IM. Ligand-protein binding affinities. In Protein Function, a Practical Approach (T. E. Creighton, Ed.). 1989; 25-35. IRL Press, Oxford**, ou encore** Ajay, Murcko MA. Computational methods to predict binding free energy in ligand-receptor complexes.J Med Chem. 1995 ; 38:4953-67**).**

Par sérum "positif" on entend un sérum contenant des anticorps produits à la suite d'une infection à *L. pneumophila,* identifiés par leur liaison avec le polypeptide (antigène) de l'invention.

On entend par "sensibilité" la proportion de malades infectés, diagnostiqués selon l'art antérieur et donnés positifs par le diagnostic selon l'invention.

On entend par "spécificité" la proportion de donneurs de sang, testés comme témoins, soumis au diagnostic selon l'invention et donnés négatifs par le diagnostic selon l'invention.

On entend par "amorces spécifiques" de courtes séquences nucléotidiques capables de s'hybrider de façon spécifique, grâce à la complémentarité des bases, sur le brin d'ADN ou sur son brin complémentaire.

On entend par "kit de diagnostic" un ensemble contenant au moins l'un des produits selon l'invention (polypeptide, polynucléotide, anticorps) et un diluant convenable, rassemblés dans un contenant approprié fait dans un matériau adapté. Ce contenant peut rassembler les différents moyens complémentaires nécessaires au test sérologique (par exemple, des réactifs marqués, des tampons, des solutions qui contiennent des ions adaptés, etc...) ainsi que les instructions requises pour réaliser le test.

La présente invention répond aux objectifs qu'elle s'est fixés plus haut en apportant un nouveau polynucléotide et un nouveau polypeptide pour le diagnostic sérologique d'infections à *L. pneumophila.*

L'invention a pour objet l'utilisation, *in vitro,* de la protéine de séquence ID SEQ N°2, d'un fragment ou d'un variant de cette protéine, de cellules hôtes comprenant des vecteurs incluant un polynucléotide codant pour ladite protéine ou un variant de ladite protéine, d'anticorps immunospécifique pour ladite protéine dans le domaine du diagnostic *in vitro* de *L. pneumophila.*

L'invention a également pour objet un kit de diagnostic comprenant :
**·** au moins un polypeptide selon l'invention, ou
**·** au moins un polynucléotide codant pour ledit polypeptide selon l'invention, ou
• au moins un anticorps immunospécifique dudit polypeptide selon l'invention.

L'invention a également pour objet une composition pharmaceutique (ou vaccin), comprenant, comme principe actif :
- au moins un polypeptide selon l'invention, ou
- au moins un polynucléotide codant pour ledit polypeptide selon l'invention.

### Utilisation de polypeptides

La présente invention a pour objet l'utilisation - pour détecter, *in vitro,* dans des échantillons biologiques, la présence d'anticorps produits à la suite d'une infection à *L. pneumophila -* du polypeptide de séquence d'acides aminés ID SEQ N°2 (appelée protéine 5E2), codée par la séquence polynucléotidique ID SEQ N°1.

La présente invention vise également l'utilisation d'un polypeptide comprenant :
a) un fragment de la séquence d'acides aminés ID SEQ N°2 ayant la même fonction que ladite séquence, ou
b) une séquence d'acides aminés ayant au moins 60% d'identité, de préférence au moins 80% d'identité, et mieux au moins 90% d'identité, avec la séquence d'acides aminés ID SEQ N°2, ou avec le fragment de séquence défini sous a), et ayant la même fonction que ladite séquence.

Ainsi, des polypeptides conformes à l'invention peuvent comprendre des variants ou des fragments de la séquence d'acides aminés ID SEQ N°2.

Les échantillons biologiques testés peuvent être du sang, de l'urine, de la salive, du liquide de ponction de sérologie (par exemple liquide céphalo-rachidien, liquide pleural ou liquide articulaire) ou l'un de leurs constituants (par exemple, le sérum).

Le diagnostic *in vitro* d'une infection à *L. pneumophila* est effectué, par exemple, par des tests classiques de réactions immunologiques, tels que ELISA ou Western Blot. Ces tests utilisent le polypeptide selon l'invention qui vient se fixer, de manière spécifique, aux anticorps sériques contre *L. pneumophila* présents dans les échantillons biologiques.

Grâce aux polypeptides définis ci-dessus, l'invention permet la détection d'anticorps naturellement produits lors d'infections à *L. pneumophila.* L'invention vise aussi l'utilisation des polypeptides selon l'invention, pour détecter, périodiquement, *in vitro*, les anticorps dirigés contre *L. pneumophila* et ainsi suivre l'évolution de la pathologie et de l'effet d'un traitement appliqué à un patient.

### Utilisation de vecteurs d'expression et de cellules hôtes

La présente invention a aussi pour objet l'utilisation du polypeptide selon l'invention préparé par culture d'une cellule hôte comprenant un vecteur recombinant ayant, inséré, un polynucléotide codant pour ledit polypeptide.

De nombreux systèmes d'expression peuvent être utilisés comme, par exemple, les chromosomes, les épisomes, les virus dérivés. Plus particulièrement, les vecteurs recombinants utilisés peuvent être dérivés de plasmides bactériens, de transposons, d'épisome de levure, d'éléments d'insertion, d'éléments chromosomiques de levures, de virus tels que les *baculovirus,* les *papillonna virus* comme SV40, les *vaccinia virus,* les adénovirus, les *fox pox virus,* les *pseudorabies virus,* les rétrovirus.

Ces vecteurs recombinants peuvent également être des dérivés de cosmides ou de phagemides. La séquence polynucléotidique peut être insérée dans le vecteur recombinant d'expression par les méthodes bien connues de l'homme du métier.

Le vecteur recombinant peut comprendre des séquences polynucléotidiques de contrôle de la régulation de l'expression du polynucléotide ainsi que des séquences polynucléotidiques permettant l'expression et la transcription d'un polynucléotide selon l'invention et la traduction d'un polypeptide selon l'invention, ces séquences étant choisies en fonction des cellules hôtes mises en oeuvre.

L'introduction du vecteur recombinant dans une cellule hôte peut être effectuée selon les méthodes bien connues de l'homme du métier telles que la transfection par phosphate de calcium, la transfection par lipides cationiques, l'électroporation, la transduction ou l'infection.

Les cellules hôtes peuvent être, par exemple, des cellules bactériennes, telles que des cellules de streptocoques, de staphylocoques, d'*Escherichia coli* ou de *Bacillus subtilis ;* des cellules de champignons, telles que des cellules de levure et des cellules d'*Aspergillus* ; des cellules de *Streptomyces* ; des cellules d'insectes, telles que des cellules de *Drosophilia S2* et de *Spodoptera Sf9* ; des cellules animales, telles que les cellules CHO, COS, HeLa, C127, BHK, HEK 293 ou encore des cellules végétales.

Le polypeptide peut être purifié à partir des cellules hôtes, selon les méthodes bien connues de l'homme du métier, telles que la précipitation à l'aide d'agents chaotropiques comme les sels, en particulier le sulfate d'ammonium, l'éthanol, l'acétone ou l'acide trichloroacétique, ou de moyens tels que l'extraction à l'acide, la chromatographie échangeuse d'ions, la chromatographie sur phosphocellulose, la chromatographie par interaction hydrophobe, la chromatographie d'affinité, la chromatographie sur hydroxylapatite ou les chromatographies d'exclusion.

### Utilisation de polynucléotides

La présente invention a également pour objet l'utilisation - pour détecter, *in vitro,* dans des échantillons biologiques, la présence d'anticorps produits à la suite d'une infection à *L. pneumophila* - d'un polynucléotide comprenant la séquence polynucléotidique ID SEQ N°1, codant pour le polypeptide ID SEQ N°2.

L'invention vise également l'utilisation d'un polynucléotide comprenant :
a) un fragment de la séquence ID SEQ N°1 et ayant la même fonction que ladite séquence, ou
b) une séquence polynucléotidique ayant au moins 60% d'identité, de préférence au moins 80% d'identité, et mieux au moins 90% d'identité, avec la séquence polynucléotidique ID SEQ N°1, ou avec le fragment de séquence tel que défini sous a), et ayant la même fonction que ladite séquence, ou
c) une séquence polynucléotidique complémentaire à la séquence polynucléotidique ID SEQ N°1 ou au fragment de séquence défini sous a) ou à la séquence définie sous b).

Ainsi, des polynucléotides conformes à l'invention peuvent comprendre des variants ou des fragments de la séquence polynucléotidique ID SEQ N°1.

Les polynucléotides de l'invention peuvent être obtenus par les méthodes standard de synthèse d'ADN ou d'ARN.

Les polynucléotides conformes à l'invention peuvent également comprendre des séquences polynucléotidiques comme les séquences 5' et/ou 3' non-codantes, telles que, par exemple, des séquences transcrites, des séquences non-traduites, des séquences signal d'épissage, des séquences polyadénylées, des séquences de liaison avec des ribosomes ou encore des séquences qui stabilisent l'ARNm.

### Utilisation des anticorps selon l'invention

L'invention vise également l'utilisation d'anticorps - pour détecter, *in vitro,* dans des échantillons biologiques, la présence d'antigènes issus d'une infection par *L. pneumophila -* lesdits anticorps étant immunospécifiques pour un polypeptide selon l'invention.

L'invention vise, en outre, l'utilisation d'anticorps selon l'invention, pour détecter, périodiquement, *in vitro,* les antigènes de *L. pneumophila* et ainsi suivre l'évolution de la pathologie et de l'effet d'un traitement appliqué à un patient.

Les anticorps immunospécifiques peuvent être obtenus par administration d'un polypeptide selon l'invention, d'un de ses fragments, d'un analogue ou d'un fragment épitopique ou d'une cellule exprimant ce polypeptide, à un mammifère, de préférence non humain, selon les méthodes bien connues de l'homme du métier.

Pour la préparation d'anticorps monoclonaux, on peut utiliser des méthodes usuelles de production d'anticorps, à partir de lignées cellulaires, telles que la technique des hybridomes, la technique des triomes, la technique des hybridomes de cellules B humaines et la technique des hybridomes EBV.

D'autres molécules, notamment protéines, suffisamment spécifiques peuvent remplacer les anticorps dans ces utilisations. Ces molécules sont, par exemple, d'autres molécules immunospécifiques comme les récepteurs de cellules T (TCR) **(**Li et al. 2005. Directed evolution of human T-cell receptors with picomolar affinities by phage display. Nat Biotechnol. 23:349-54**).** Ces molécules peuvent également être des protéines sélectionnées pour leur fixation spécifique des polypeptides selon l'invention, par exemple, par évolution dirigée (voir par exemple Conrad et Scheller. 2005. Considerations on antibody-phage display methodology. Comb Chem High Throughput Screen. 8:117-26**).**

### Isotypes d'anticorps et affinité

Les immunoglobulines (ou anticorps) sont constituées de deux chaînes polypeptidiques différentes : deux chaînes légères (L) d'isotypes [kappa] ou [lambda], et deux chaînes lourdes (H) d'isotypes [gamma], [alpha], [mu], [delta] ou [epsilon]. Ces quatre chaînes sont liées covalemment par des ponts disulfures. Les deux types de chaînes H ou L possèdent des régions qui contribuent à la fixation des antigènes et sont très variables d'une immunoglobuline à une autre. Ces régions déterminent l'affinité des anticorps pour leur ligand.

Les isotypes des chaînes lourdes permettent de définir la classe de l'immunoglobuline ; il existe donc 5 isotypes d'anticorps (IgA, IgM, IgD, IgE et IgG). Des sous-classes, par exemple IgG1, IgG2, IgG3 et IgG4, sont définies par des différences de séquence de la chaîne lourde. Les différentes classes d'immunoglobulines ont des propriétés physicochimiques propres et leur synthèse dépend directement des phases et des niveaux d'activation de la réponse immunitaire.

Chez l'homme, les IgG représentent la principale classe d'immunoglobulines : leur concentration sérique chez l'adulte varie de 8 à 16 g/l. Leur demi-vie plasmatique est d'environ 3 semaines.

Les IgA constituent la deuxième classe d'immunoglobulines sériques, après les IgG, en terme de concentration (2 à 4 g/l). En revanche, elles représentent la classe prépondérante des immunoglobulines dans les sécrétions (sécrétions respiratoires, salivaires, digestives..., lait, colostrum, larmes). Sur le plan structural, les IgA ont la particularité de se présenter sous plusieurs formes moléculaires :
- dans le sérum, les IgA peuvent se présenter sous forme de monomères (forme prépondérante) ou sous forme de dimères associés à une chaîne J (chaîne de jonction). La chaîne J est un peptide riche en cystéine de 137 acides aminés (poids moléculaire : 15 000 Da), d'origine plasmocytaire, la sous-classe IgA1 étant prépondérante dans le sérum ;
- dans les sécrétions, les IgA, appelées IgA sécrétoires, sont sous forme de dimères ; elles sont donc associées à une chaîne J, mais elles comportent également un composant sécrétoire. Les IgA produites par les lymphocytes B sont captées par les cellules épithéliales, par un récepteur (polyIgR) situé au pôle basal de la cellule. C'est pendant le transfert de l'IgA à travers la cellule épithéliale, vers le pôle apical de la cellule, que le composant sécrétoire (poids moléculaire : 70 000 Da), ou pièce sécrétoire, est ajouté. Le rôle de la pièce sécrétoire est de protéger les IgA sécrétoires vis-à-vis des enzymes protéolytiques présents dans les sécrétions.

Comme les IgA, les IgM peuvent se présenter sous 2 formes moléculaires distinctes :
- une forme monomérique : c'est la forme sous laquelle les IgM sont synthétisées et insérées dans la membrane du lymphocyte B ;
- une forme pentamérique : c'est la forme sous laquelle les IgM sont sécrétées. Les 5 monomères de base sont reliés par des ponts disulfures. De plus, une chaîne J relie l'extrémité de 2 monomères.

Les IgD représentent moins de 1 % des immunoglobulines sériques. Elles sont habituellement coexprimées avec les IgM à la surface des lymphocytes B où elles semblent jouer un rôle de récepteur pour les antigènes.

Les IgE ne sont présentes, chez un sujet normal, qu'à l'état de traces.

### Cinétique d'apparition des anticorps et affinité

La cinétique d'apparition d'anticorps spécifiques et d'un isotype donné est aujourd'hui encore mal comprise. D'une façon générale les cellules B, dites naïves, synthétisent différentes IgM qui constituent un large répertoire de molécules capables de fixer des antigènes **(**Steven A. Frank., Immunology and Evolution of Infectious Disease. (2002), Princeton University Press, Princeton, USA**).** Ainsi lors de la première exposition à un antigène, celui-ci va se fixer avec une faible affinité à différents IgM du système immunitaire. Cette interaction va néanmoins stimuler la division de la cellule B naïve correspondante. La division est d'autant plus rapide que l'affinité de l'IgM est forte, ce qui permet une sélection initiale des meilleurs clones. De plus, lors de la division, les réarrangements génétiques permettent l'augmentation de l'affinité des anticorps. La région constante des immunoglobulines change également afin de produire des IgG dans le système circulatoire et des IgA à la surface des muqueuses **(**Steven et Frank 2002. Immunology and Evolution of Infectious Disease. Princeton University Press, Princeton, USA**).**

### Utilisation des isotypes d'anticorps selon l'invention

L'homme de l'art peut donc déduire de ce qui est explicité ci-dessus que la présence d'une ou plusieurs classes d'anticorps et l'affinité particulière des anticorps ont différentes implications d'un point de vue du diagnostic médical dans la différentiation des infections récurrentes ou non, d'une infection active, aiguë, chronique, latente, et/ou récente.

### Les vaccins

La présente invention concerne également une composition pharmaceutique, utilisable comme vaccin, renfermant à titre de principe actif au moins un polypeptide, polynucléotide, vecteur recombinant ou cellule hôte selon l'invention, et un excipient pharmaceutiquement acceptable (par exemple, une solution stérile aqueuse ou non, pouvant contenir un antioxydant ou un tampon ou un soluté rendant la composition isotonique pour les fluides corporels).

### Les kits

L'invention a également pour objet des kits de diagnostic *in vitro* comprenant, d'une part :
- au moins un polypeptide conforme à l'invention, ou
- au moins un polynucléotide codant pour ledit polypeptide, ou
- au moins un anticorps conforme à l'invention,
et, d'autre part, au moins un diluant (par exemple, un tampon, une solution saline...).

### Partie expérimentale

### A) Protocole d'obtention de l'antigène

### A.1. Clonage de la séquence codant pour le polypeptide ID SEQ N°2

Le gène codant pour la séquence du polypeptide, qui est un antigène, est obtenu par amplification par PCR à partir de l'ADN génomique des bactéries *L. pneumophila* (souche Philadelphia-1, ATCC 33152), en utilisant des amorces spécifiques de la séquence ID SEQ N°1. Ces amorces sont choisies par l'homme de l'art de façon à encadrer la séquence d'ADN ID SEQ N°1 et à l'amplifier spécifiquement.

Le fragment correspondant, ainsi amplifié, est cloné dans un vecteur selon des techniques classiques bien connues de l'homme du métier. Ce vecteur permet la production des protéines clonées sous contrôle d'un promoteur inductible par l'isopropyl-thiogalactoside (IPTG). La protéine clonée correspond au polypeptide ID SEQ N°2.

### A.2. Expression de la protéine

Une souche d'*Escherichia coli* est transformée par les vecteurs d'expression précédemment décrits. Les bactéries sélectionnées sont mises à cultiver une nuit à 30°C sous agitation, dans 30 ml de milieu Luria Bertani **(**LB, J. Miller, "A short Course in Bacterial Genetics", Cold Spring Harbor Laboratory Press, 1992**)** contenant de l'ampicilline à une concentration finale de 100 µg/ml. Le lendemain, la culture est diluée au 1/50^{ème} dans un volume final de 1 litre de milieu LB additionné d'ampicilline à une concentration finale de 100 µg/ml et est incubée à 30°C sous agitation. Lorsque la turbidité de la culture atteint une valeur d'absorbance à 600 nm (en abrégé, A600) d'environ 0,7, la production de la protéine est induite par l'isopropyl-thiogalactoside (en abrégé, IPTG) à une concentration finale de 0,1 mM. Les bactéries sont récoltées par centrifugation (10 minutes à 1400xg et à 4°C) lorsque la turbidité de la culture atteint une A600 d'environ 1,5.

### A.3. Purification de la protéine 5E2 (ID SEQ N°2)

Après centrifugation, les cellules sont remises en suspension dans un tampon Tris-HCl 20 mM à pH 8,0 contenant du saccharose à 0,5 mM, puis traitées par du lysozyme (0,2 g/1) en présence d'acide éthylènediaminotétraacétique (EDTA) 12,5 mM, de DNase, RNase et PMSF. La suspension est incubée 30 minutes à 4°C, puis centrifugée 10 minutes à 4°C à 15500xg. Le culot est congelé à -20°C pendant au moins une nuit.

Après décongélation, les bactéries sont reprises dans un tampon phosphate 50 mM (plus NaCl 0,5 M) à pH 8,0, puis soniquées 4 fois 20 secondes dans la glace. Après centrifugation à 15500xg à 4°C pendant 30 minutes, le surnageant est filtré sur membrane de porosité 0,22 µm.

La protéine peut être (mais pas nécessairement) purifiée par chromatographie. Dans ce cas, le filtrat est déposé sur une colonne de chromatographie choisie par l'homme du métier, par exemple une colonne d'affinité ou une colonne d'échange d'ions ou encore des colonnes d'interaction hydrophobes ou colonnes d'exclusion stérique. En général, après le dépôt de l'échantillon, une étape de chromatographie nécessite (mais pas nécessairement) un lavage par du tampon, après quoi la protéine fixée est éluée par un tampon contenant un compétiteur de l'interaction (par exemple, le chlorure de sodium ou le sulfate d'ammonium sont très employés). Les fractions contenant la protéine sont rassemblées et la pureté de la protéine est évaluée. En général, plusieurs étapes de chromatographie sur des colonnes différentes (ou non) sont nécessaires pour obtenir la pureté suffisante. Dans certaines colonnes, la protéine n'est pas fixée mais retenue transitoirement dans la colonne, par exemple dans une colonne de gel filtration.

Les fractions contenant la protéine 5E2 purifiée sont rassemblées et du glycérol est ajouté jusqu'à une concentration finale de 20%. La protéine purifiée est alors stockée à -20°C jusqu'à son utilisation dans les tests.

La concentration de la protéine est déterminée spectrophotométriquement à partir des coefficients d'absorption calculés par la méthode de Pace **(**Pace et al. 1995. How to measure and predict the molar absorption coefficient of a protein. Protein Science 4, 2411-2423**).** La pureté est vérifiée par analyse par électrophorèse SDS-PAGE et par spectrométrie de masse.

### B) Test de diagnostic in vitro

Il a été utilisé des sérums provenant de patients ayant eu une infection documentée à *Legionella pneumophila* (collection du laboratoire). L'infection a pu être mise en évidence soit par l'isolement/culture des bactéries à partir de prélèvements broncho-pulmonaires, soit par une séroconversion, ou encore par un test urinaire positif.

Les sérums témoins correspondaient à des sérums de donneurs de sang (collection du laboratoire).

La fixation, sur les protéines recombinantes purifiées (obtenue comme décrit précédemment), des anticorps présents dans les sérums peut être évaluée soit par des tests en Western Blot soit par la technique ELISA.

### Exemple B1 : Protocole de test pour les polypeptides selon l'invention en ELISA

La fixation des anticorps présents dans les sérums a été évaluée par des tests ELISA. Les plaques ELISA sont laissées pendant une nuit à 4°C en présence de 0,5 µg d'antigène purifié dans du "tampon salin phosphate" (PBS). Après quatre lavages par du PBS contenant du polyoxyéthylène sorbitan (Tween) à 0,05%, les plaques sont saturées une heure à 37°C par du PBS contenant 4% d'albumine de sérum bovin (BSA) (250 µl par puits). Quatre nouveaux lavages sont réalisés, puis 100 µl de chaque sérum à tester, à la dilution adéquate dans du tampon PBS-Tween, sont ajoutés dans chaque puits. La plaque est ensuite laissée à 25°C pendant 30 minutes. Après quatre nouveaux lavages, des anticorps (secondaires) de chèvre, marqués à la phosphatase alcaline (par exemple 170-6462, Biorad), anti-immunoglobulines G ou A humaines sont ajoutés et incubés pendant 30 minutes à 25°C après avoir été dilués selon le protocole du fournisseur dans du tampon PBS contenant 4 % de BSA. Quatre nouveaux lavages sont réalisés puis 100 µl de substrat (phosphate de p-nitrophényle, pNPP, par exemple A-3469, Sigma) sont ajoutés. L'absorbance à 405 nm de chacun des puits est mesurée après une incubation de 1 heure à 37°C.

Sont considérés comme "positifs" en ELISA, les sérums identifiés par leur liaison avec les polypeptides (antigènes) de l'invention.

### Exemple B2 : Protocole de test pour les polypeptides selon l'invention en Western Blot

La fixation éventuelle des anticorps présents dans les sérums peut également être évaluée par des tests en Western Blot, par exemple, sur des extraits totaux de bactéries produisant le polypeptide selon l'invention ou sur la protéine purifiée selon l'invention.

Pour cela, la membrane de nitrocellulose sur laquelle les protéines de l'extrait sont transférées est saturée pendant 30 minutes à l'aide d'une solution de "tampon salin phosphate" (PBS) contenant 3% de lait demi-écrémé. Après trois lavages par du PBS contenant du polyoxyéthylène sorbitan (Tween) à 0,05%, la membrane est mise en présence du sérum testé, à la dilution adéquate dans du tampon PBS contenant 3% de lait demi-écrémé, pendant 45 minutes. Après trois nouveaux lavages, des anticorps (secondaires) de chèvre anti-immunoglobulines G, M ou A ou simultanément anti-immunoglobulines G et/ou A et/ou M humaines, marqués à la phosphatase alcaline (par exemple 170-6462, Biorad), sont ajoutés pendant une période de 30 minutes après dilution, selon le protocole du fournisseur, dans du tampon PBS contenant 3% de lait demi écrémé. Trois nouveaux lavages sont réalisés puis la membrane est incubée en présence de phosphate de 5-bromo-4-chloro-3-indolyle et de nitroblue tetrazolium selon les indications du fournisseur. Un résultat "positif" correspond à la fixation de l'anticorps anti-immunoglobuline humaine à un complexe composé du polypeptide selon l'invention fixé à la membrane et de l'anticorps sérique humain le reconnaissant spécifiquement, ce qui se traduit par une coloration localisée au niveau dudit complexe.

### C) Résultats et interprétation

Des résultats types sont présentés dans l'exemple ci-après en utilisant des anti-IgA comme anticorps secondaires. Des résultats similaires sont obtenus en utilisant des anti-anticorps spécifiques d'autres isotypes d'immunoglobulines comme anticorps secondaires.

**Tableau 1 : Résultats (ELISA) obtenus en utilisant des anti-IgA comme anticorps secondaires**

| | Polypeptide 5E2 |
|---|---|
| Proportion de sérums "positifs" parmi les 69 sérums de malades infectés par *L. pneumophila* diagnostiqués selon l'art antérieur et soumis au diagnostic selon l'invention | 35,6% |
| Proportion de sérums "négatifs" parmi les 96 sérums de donneurs de sang testés comme témoins | 95,8% |

D'après les résultats, on constate que, par le test ELISA, on identifie *in vitro* des infections à *Legionella pneumophila,* grâce à l'antigène 5E2 selon l'invention.

Des résultats similaires sont obtenus par le test de Western blot.

En conclusion, la présente invention décrit un polypeptide choisi, quant à sa sensibilité et à sa spécificité, parmi un très grand nombre, pour une utilisation comme sonde (antigène) dans des tests sérologiques lors d'infections naturelles à *L. pneumophila.*

## Revendications

1. Utilisation du polypeptide de séquence d'acides aminés ID SEQ N°2 pour détecter, *in vitro,* dans des échantillons biologiques, la présence d'anticorps produits à la suite d'une infection à *Legionella pneumophila.*

2. Utilisation pour détecter, *in vitro,* dans des échantillons biologiques, la présence d'anticorps produits à la suite d'une infection à *Legionella pneumophila,* d'un polypeptide comprenant :
a) un fragment de la séquence d'acides aminés telle que définie selon la revendication 1 et ayant la même fonction que ladite séquence, ou
b) une séquence d'acides aminés ayant au moins 60% d'identité avec la séquence d'acides aminés telle que définie selon la revendication 1 ou avec un fragment de séquence tel que défini sous a), et ayant la même fonction que ladite séquence.

3. Utilisation selon la revendication 1 ou 2, **caractérisée en ce que** le polypeptide est préparé par culture d'une cellule hôte comprenant un vecteur recombinant ayant, inséré, un polynucléotide codant pour ledit polypeptide et par isolement dudit polypeptide du milieu de culture.

4. Utilisation selon la revendication 3, quand elle dépend de la revendication 1, **caractérisée en ce que** le polynucléotide est de séquence ID SEQ N°1.

5. Utilisation d'un polynucléotide de séquence ID SEQ N°1 pour détecter, *in vitro*, dans des échantillons biologiques, la présence d'anticorps produits à la suite d'une infection à *Legionella pneumophila.*

6. Utilisation pour détecter, *in vitro,* dans des échantillons biologiques, la présence d'anticorps produits à la suite d'une infection à *Legionella pneumophila,* d'un polynucléotide comprenant
a) un fragment de la séquence telle que définie selon la revendication 5 et ayant la même fonction que ladite séquence, ou
b) une séquence polynucléotidique ayant au moins 60% d'identité avec la séquence polynucléotidique telle que définie selon la revendication 5 ou avec un fragment de séquence tel que défini sous a), et ayant la même fonction que ladite séquence, ou
c) une séquence polynucléotidique complémentaire à une des séquences polynucléotidiques telles que définies selon la revendication 5 ou selon la revendication 6a) ou 6b).

7. Utilisation d'anticorps pour détecter, *in vitro,* dans des échantillons biologiques, la présence d'antigènes issus d'une infection par *Legionella pneumophila,* **caractérisée en ce que** lesdits anticorps sont immunospécifiques pour un polypeptide tel que défini dans les revendications 1 à 3.

8. Kit de diagnostic pour l'utilisation selon l'une quelconque des revendications 1 à 6, **caractérisé en ce qu'**il comprend au moins l'un desdits polypeptides ou l'un desdits polynucléotides codant pour lesdits polypeptides associé à au moins un diluant.

9. Kit de diagnostic pour l'utilisation selon la revendication 7, **caractérisé en ce qu'**il comprend au moins l'un desdits anticorps associé à au moins un diluant.

10. Composition pharmaceutique, notamment vaccin, comprenant, à titre de principe actif, au moins :
a) un polypeptide de séquence d'acides aminés ID SEQ N°2, ou
b) un polypeptide dont la séquence est un fragment de la séquence d'acides aminés telle que définie sous a) et ayant la même fonction que ladite séquence, ou
c) un polypeptide dont la séquence est une séquence d'acides aminés ayant au moins 60% d'identité avec la séquence d'acides aminés telle que définie sous a) ou avec le fragment de séquence tel que défini sous b), et ayant la même fonction que ladite séquence,
ainsi qu'un excipient pharmaceutiquement acceptable.

11. Composition pharmaceutique, notamment vaccin, comprenant, à titre de principe actif, au moins :
a) un polynucléotide de séquence ID SEQ N°1 ou
b) un polynucléotide dont la séquence est un fragment de la séquence polynucléotidique telle que définie sous a) et ayant la même fonction que ladite séquence, ou
c) un polynucléotide dont la séquence est une séquence polynucléotidique ayant au moins 60% d'identité avec la séquence polynucléotidique telle que définie sous a) ou avec un fragment de la séquence telle que définie sous b), et ayant la même fonction que ladite séquence, ou
d) un polynucléotide dont la séquence est une séquence polynucléotidique complémentaire à l'une des séquences polynucléotidiques telles que définies sous a), b) ou c)
ainsi qu'un excipient pharmaceutiquement acceptable.
